# EUROPEAN PATENT SPECIFICATION

(11) **EP 4 031 224 B1**
(45) Date of publication and mention of the grant of the patent: **02.12.2026**
(21) Application number: 21876571.7
(22) Date of filing: 01.10.2021
(51) Int. Cl.: A61M 25/01, A61M 25/00

(54) **STYLET WITH IMPROVED THREADABILITY**
MANDRIN MIT VERBESSERTER EINFÄDELBARKEIT
STYLET À CAPACITÉ DE VISSAGE AMÉLIORÉE

(30) Priority: 01.10.2020 US 202063086583 P
(43) Date of publication of application: 27.07.2022
(73) Proprietor: Teleflex Medical LLC, Morrisville, North Carolina 27560 (US)
(72) Inventor: HORST, Benjamin, Lititz, Pennsylvania 17543 (US); AMAN, Michael, Sinking Spring, Pennsylvania 19608 (US); SPINKA, Mark, Jenkintown, Pennsylvania 19046 (US)
(74) Representative: Carpmaels & Ransford LLP
(86) International application number: PCT/US2021/053118
(87) International publication number: WO 2022/072795

(56) References cited:
- US-A1- 2004 102 829
- US-A1- 2006 276 868
- US-A1- 2010 004 662
- US-A1- 2010 179 562
- US-A1- 2015 105 721
- US-B2- 8 728 075

## Description

### Technical Field

The present disclosure relates generally to stiffening wires or stylets for placement of vascular catheters, and more particularly to low or non-torqueable stiffening wires or stylets with improved threadability.

### Background

A PICC (PERIPHERALLY Inserted Central Venous Catheter) is a catheter to be inserted into a vein of the upper arm and threaded to the Superior Vena Cava (SVC). The PICC provides access to the large central veins near the heart, generally to administer medications or liquid nutrients. The PICC can help avoid the pain of frequent needle sticks and reduce the risk of irritation to the smaller veins in your arms

US 2010/01719562 A1 discloses a stylet made of superelastic nitinol, that straightens when inserted into a lead and sheath but recovers its preset shape once advanced beyond the distal end of the sheath.

### Overview

The invention is defined by the independent claim. A selection of optional features of the invention is set out in the dependent claims.

Furthermore in the description references are made to certain non-SI units (inches). These are to be converted to SI units (mm) using the following factor: 1 inch = approx. 25.4 mm.

### Brief Description of the Drawings

In order that the invention may be readily understood, this invention is illustrated by way of examples in the accompanying drawings.
FIG. 1 illustrates vasculature of a human body.
FIG. 2 illustrates a perspective view of a first embodiment of a stylet according to the present invention.
FIG. 3 illustrates a side view of a distal portion of the stylet of FIG. 2.
FIG. 4 illustrates a perspective view of the distal portion of a core wire and a tubular body of the stylet of FIGS. 2-3.
FIG. 5 illustrates a side view of the distal portion of the core wire and the tubular body of the stylet of FIGS. 2-4.
FIG. 6 illustrates the distal portion of the stylet of FIGS. 2-5 in a catheter being threaded through the vasculature of the human body of FIG. 1.
FIG. 7 illustrates an isometric view of a housing member of the stylet of FIGS. 1-6.
FIG. 8A-B illustrate views of a second embodiment of a stylet according to the present invention.
FIG. 9 illustrates a side view of a third embodiment of the stylet according to the present invention.
FIGS. 10A-B illustrate views of a fourth embodiment of the stylet according to the present invention.
FIGS. 11A-B illustrate views of a fifth embodiment of the stylet according to the present invention.
FIG. 12 illustrates a side view of the stylet of FIGS. 11A-B.
FIG. 13A illustrates an isometric view of a sixth embodiment of a stylet in a catheter according to the present invention.
FIG. 13B illustrates a distal end of the stylet and catheter of FIG. 13A.
FIGS. 14A-C illustrate a first variation of the stylet of FIGS. 13A-B.
FIG. 15 illustrates a second variation of the stylet of FIGS. 13A-B.
FIG. 16 illustrates an isometric view of a seventh embodiment of a stylet in a catheter according to the present invention.
FIG. 17 illustrates an isometric view of an eighth embodiment of a pair of stylets according to the present invention.
FIG. 18 illustrates a first assembly including the pair of stylets of FIG. 17 in a catheter.
FIG. 19 illustrates a second assembly including the pair of stylets of FIG. 17 in a catheter.

The same reference numbers are used in the drawings and the following detailed description to refer to the same or similar parts.

### Detailed Description

The present invention is generally directed to a stylet providing a specific combination of bend and/or stiffness profiles that enables a catheter to be consistently threaded through anatomical curves of the vasculature into a desired location of the vasculature. The stylet of the present invention may be low or non-torqueable and allow the user to thread the stylet without needing to know the distal tip orientation (*i*.*e*., the direction the tip is pointing, "up" or "down") or control the distal tip orientation by rotating the proximal end of the stylet. More specifically, the stylet may provide a consistently favorable approach angle through the vasculature (*e*.*g*., at a confluence of the internal jugular vein, brachiocephalic vein, and/or subclavian vein) into the desired location. This approach angle may be favorable regardless of how the user inserts the catheter into the vasculature and/or rotates the proximal end of the catheter during threading. The stylet may further have a narrow segment that is more flexible or floppier than the other portions of the stylet. The floppiness of the narrow segment may enhance the deflection of the distal tip when contacting a vein wall to reduce vessel trauma. Furtherrnore, the narrow segment may improve the contact angle of the distal tip by bowing upward when the distal tip contacts the vein wall, such the distal tip points downward in a favorable direction. Commercial benefits include fewer insertion attempts, less insertion time, and reduced user frustration during insertion.

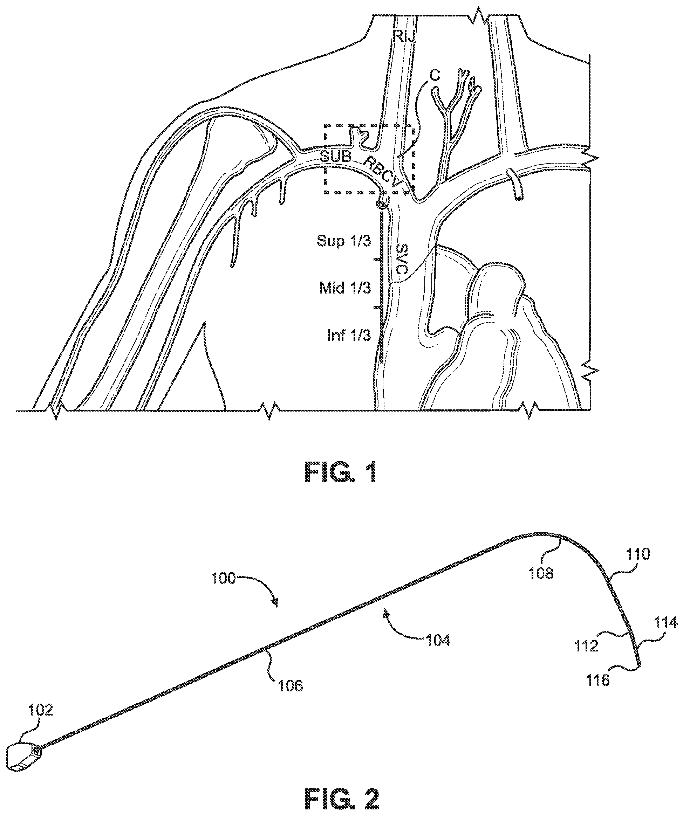

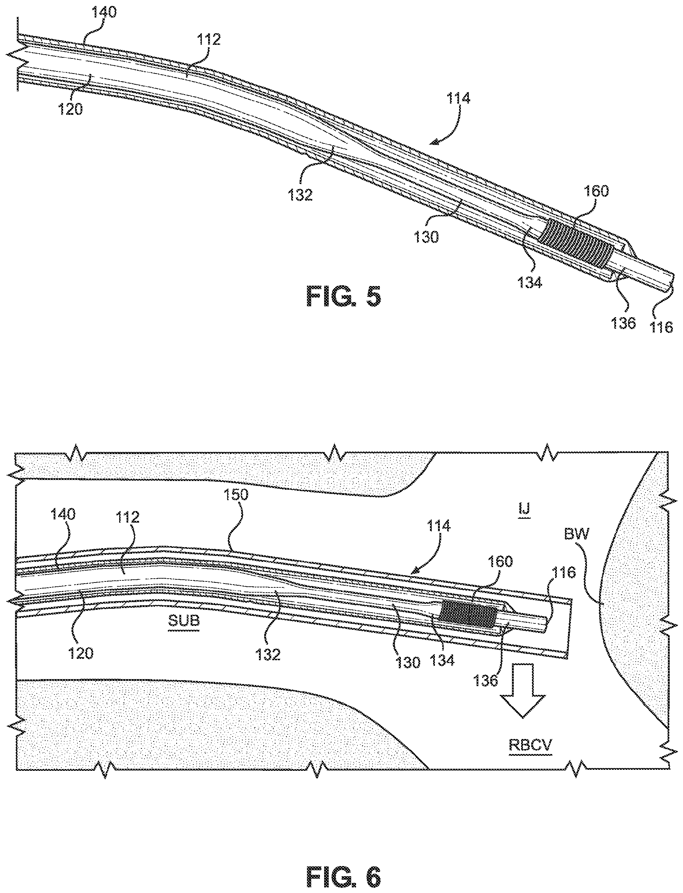

FIG. 1 illustrates a reference anatomical graphic that includes vasculature of a potential pathway through which a stylet assembled with a catheter (e.g., a peripherally inserted central venous catheter, PICC) can be threaded to the superior vena cava (SVC), according to the present invention. The stylet/PICC may enter the vasculature at a number of different venous locations (e.g., the basilic, brachial, or cephalic vein). A needle puncture may access the vasculature at the venous location of choice, an access wire may be inserted through the needle into the vasculature, a sheath may be guided over the access wire after the needle is removed. After the access wire is removed from the sheath, the stylet/PICC may then be threaded through the sheath entering the vasculature at the venous location of choice, into and through a subclavian vein (SUB), into and through a brachiocephalic vein (BCV, *e.g.,* the right brachiocephalic vein, RBCV), and ultimately into the SVC. The most common area for insertion difficulty is the area of a confluence (C) where the SUB ends and joins the internal jugular vein (13) and a BCV, as further illustrated in FIG. 6. The confluence (C) typically contains the sharpest curve in the anatomical pathway which may define a 90° curve or drop. Due to this sharp curve, the catheter may deflect the wrong way *(e.g.,* up the IJ) or get caught and not be able to be advanced at all. Although FIG. 1 and corresponding discussion is directed to insertion of the stylet through the SUB into the RBCV, the stylet of the present invention may be inserted through other pathways including through the left brachiocephalic vein (LBCV).

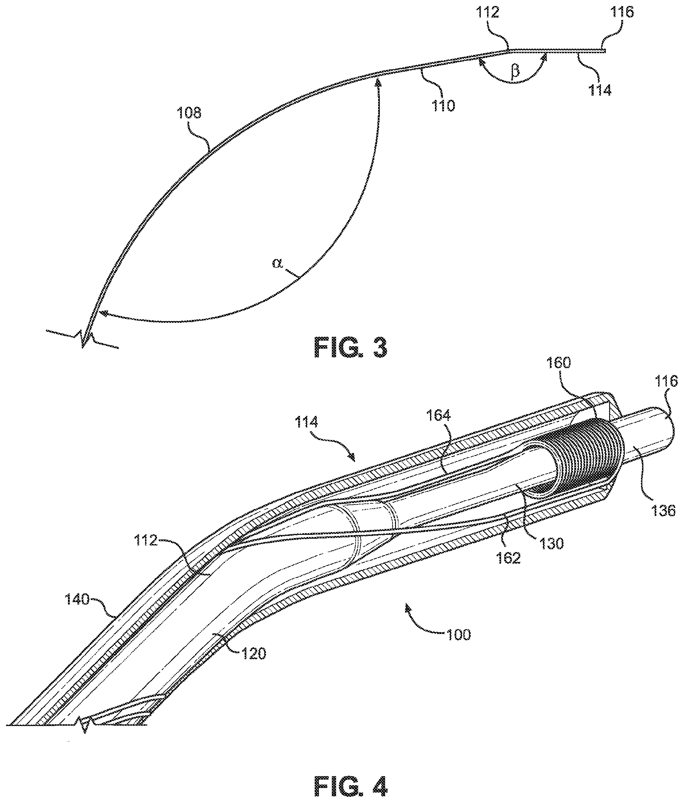

FIG. 2 illustrates an isometric view of a stylet 100 according to the present invention, and FIG. 3 illustrates a side view of a distal end of the stylet 100. As illustrated, the stylet 100 may include a housing member 102 at a proximal end of an elongated body 104 configured to be inserted through the vasculature. The elongated body 104 may include a core wire 120 and a tubular body 140 disposed around the core wire 120, as illustrated in FIGS. 4-6.

The core wire 120 may have a nominal or pre-formed shape as illustrated in FIGS. 2 and 3, including a pre-formed first curve or bend α ("body bend"). During insertion, the first bend α may be forced to elastically straighten due to the anatomical insertion pathvvay that is straighter than the pre-formed core wire. The core wire 120, when straightened, is in a state of high potential energy and will try to compress back to its nominal shape to a state of lower potential energy, akin to a stretched spring trying to return to its original unstressed condition. When the distal tip 116 reaches the confluence (C), the lowest potential energy state of the first bend α forces the distal tip 116 to point down towards the SVC. The nominal shape and rigidity of the elongated body 104 may be formed by the pre-formed shape and rigidity of the core wire 120. As illustrated, the core wire 12.0 (and elongated body 104) may have a first portion 106 that is substantially straight in the nominal state and extends distally from the housing member 102. The core wire 120 (and elongated body 104) may have a second portion 108 that has the pre-formed first curve or bend α and extends distally from the first portion 106. The core wire 120 (and elongated body 104) may have a third portion (or intermediate portion) 110 that is substantially straight in the nominal state and extends distally from the second portion 108. The core wire 120 (and elongated body 104) may have a fourth portion 112 that has a pre-formed second curve or bend β and extends distally from the third portion 110. The core wire 120 (and elongated body 104) may have a fifth portion (or distal portion) 114 that is substantially straight in the nominal state and extends distally from the fourth portion 112 to the distal tip 116. Thus, the first bend α may be proximal of the second bend β and be spaced apart by the third portion 110. As further illustrated in FIG. 3, the first bend α may define a first angle of about 15-90° (e.g., 55-70°) between α longitudinal axis of each of the first and third portions 106, 110, thus the first bend α may define a supplementary arc (as illustrated in FIG. 3) of about 90-165° (however, the angle of the first bend α is defined herein between the longitudinal axis of each of the first and third portions 106, 110, unless otherwise indicated). The second bend β may define a second angle of about 8-15° between a longitudinal axis of the third and fifth portions 110, 114, thus the second bend β may define a supplementary arc (as illustrated in FIG. 3) of about 165-172° (however, the angle of the second bend β is defined herein between the longitudinal axis of each of the third and fifth portions 110, 114, unless otherwise indicated). The angles of the first bend α and second bend β may vary depending on the stiffness of a catheter (e.g., a PICC) 150 disposed around the stylet 100. In other words, the catheter 150 may nominally be straighter and have some stiffness, thus it may straighten out the stylet 100 at the first bend α and second bend β. The stiffness of the catheter 150 may depend on the brand, material, lumen, and size. For a stiffer catheter 150, the stylet 100 may be more curved to induce a proper angle for the vasculature. Thus, the stylet 100 may be designed to produce an assembly of the stylet 100 and catheter 150 with a first portion at the first bend α, having an angle of 5-80° and a second portion at the second bend β having an angle of 3-10°, each relative to the longitudinal axis of the assembly. Thus, the assembly of the stylet 100 and the catheter 150 is of a greater angle than the anatomical reference curvature.

The second portion 108 may have an arc length of about 3-7 inches (about 7.5-17.5 cm), *e.g.,* about 5.3 inches (about 13.5 cm), the third portion 110 may have a length of about 0-3 inches (about 0-7.5 cm), the fourth portion 112 may have an arc length of about 0.2-0.5 inches (about 0.5-1.25 cm), and the fifth portion 114 may have a length of about 1-2 inches (about 2.5-5 cm). Thus, in a favorable embodiment for inserting a PICC, the first bend α may be a gradual bend at a first angle of about 60° starting about 3 inches (about 7.5 cm) from the end of the distal tip 116 and extending proximally to about 8.3 inches (about 21 cm) from the end of the distal tip 116. The second bend β may form a second angle of about 10°, for example, by bending the stylet 100 around a pin of about 0.25 inch (about 0.5 cm) diameter positioned about 1.25 inch (about 3 cm) from the end of the distal tip 116 and forming an arc length of about 0.22 inch (about 0.5 cm).

Therefore, the first angle of the first bend α (between the first and third portions 106, 108) may be substantially greater than the second angle of the second bend β (between third and fifth portions 110, 114). For example, the difference between the first angle of the first bend α and the second angle of the second bend β may be at least 40°. Furthermore, the arc length of the second portion 108 (and first bend α) may be substantially greater than the are length of the fourth portion 112 (and second bend β). For example, the difference between the arc length of the second portion 108 (and first bend α) and the arc length of the fourth portion 112 (and second bend β) may be at least 4 inches (about 10 cm), and the first bend α and the second bend β may entirely be within 10 inches (25 cm) of the distal tip 116. Thus, the second bend β may have a proximity to the distal tip 116 such that when the distal tip 116 is at the confluence (C), the second bend β is in an area of reference anatomy (upstream of/towards the insertion site) that contains some curvature.

The first bend α may correspond to a body curve and the second bend β may correspond to a tip curve, and the first and second bends α, β may be in the same plane and same direction (to prevent counteracting of the bends α, β). The first bend α may be configured to self-orient the stylet 100 in the anatomy, and the second bend β may be configured for the distal tip 116 to approach the difficult curve locations of the anatomy at a favorable angle when the first bend α is self-oriented. Thus, the first and second bends α, β may increase the user's ability to thread the stylet 100 into the desired position, without visualization of the vessel or steering the stylet 100. For example, as illustrated in FIG. 6, the first and second bends α, β may be configured to automatically keep the distal tip 116 at an approach angle directed toward a lower portion of a back wall (BW) of the vasculature increasing the chance that the distal tip 116 points down towards the SVC. Additionally, the angle of the distal tip 116 reduces the chances that the distal tip 116 hits normal to the back wall ensuring that the distal tip 116 bows in a predictable direction and does not start to loop back onto itself or "tent". Furthermore, the distal tip 116 hitting at the favorable angle may cause the distal tip 116 to glance off the back wall (BW) of the vein and thread in the right direction down the RBVC without the user encountering too much resistance and therefore reducing trauma to the back wall (BW).

The stylet 100 may be self-orienting in that manual rotation of the proximal housing member 102 does not substantially transmit torque to the distal tip 116 enough to cause a consistent rotation. Thus, the core wire 120 may be low or non-torqueable. For example, the user may rotate the proximal housing member 102 a number of times before there is significant rotational movement translated to the distal tip 116, then the distal tip 116 may axially rotate around fully and quickly, and go back to a favorable location.

Thus, the first bend α may be configured to automatically align the stylet 100 with the curvature of the vasculature during insertion in order for the distal tip 116 to point "down" into the RBVC since the first and second bends α, β are in the same plane and same direction. The distal tip 116 may always points down when the distal tip 116 enters, for example, the confluence (C) of the vessels where PICCs have the most difficulty in navigating through the tight curvature. The stylet 100 may be non-torqueable and non-steerable removing control of the orientation of the distal tip 116 from the user and enabling the stylet 100 to automatically find the correct orientation in the vasculature no matter what orientation the stylet 100 is initially inserted and/or how the stylet 100 is rotated during insertion. Furthermore, in some embodiments, the stylet 100 may include a torque-limiting member on a proximal end of the core wire 120 that further reduces the ability of the user to transmit torque (*e*.*g*., a torque limiting ground section).

As further illustrated in FIGS. 4-6, the fifth portion 114 (including the distal tip 116) may have a narrow segment 130 that is more flexible or floppier than at least one or all of the proximal portions 106-112 of the stylet. The floppiness of the distal tip 116 may allow the distal tip 116 to deflect when contacting a vein wall without causing vessel trauma. Furthermore, the narrow segment 130 may improve the contact angle of the distal tip 116 by bowing upward when the distal tip 116 contacts the vein wall, such that the distal tip 116 points downward toward the RBCV. The narrow segment 130 may be straight and have a constant width or diameter. The fifth portion 114 may have a short length to provide favorable deflection mechanics. The proximal portions 106-112 of the core wire 120 need to have sufficient stiffness (greater than the narrow segment 130) such that the shape of the core wire 120 influences the stylet 100 after assembly and the stylet 100 does not buckle too easily during insertion. Thus, the stylet 100 has a stiffness profile such that the narrow segment 130 of the fifth portion 114 may be the first location along the length of the core wire 120 to buckle which may improve the insertion angle of the distal tip 116 and maintain pushability of the assembly. If the proximal portions 106-112 of the core wire 120 buckle prior to the narrow segment 130 of the fifth portion 114, any longitudinal pushing force applied by the user may no longer transmitted to the distal tip 116 preventing the stylet 100 from advancing forward.

The narrow segment 130 of the fifth portion 114 may have a reduced width or diameter and be formed by a proximal taper 132 at a proximal end reducing the width or diameter of the stylet 100 from the fourth portion 112 and/or a distal taper 134 at a distal end increasing the width or diameter of the stylet 100 to a distal segment 136. The narrow segment 130 may have a rounded and/or flattened cross-section. Thus, the distal taper 134 may connect the narrow segment 130 to the distal segment 136, which connects to the distal tip 116. In some embodiments, the distal segment 136 and the distal tip 116 may have a width or diameter greater than a width or diameter of the narrow segment 1 30 and less than a width or diameter of the at least one of the portions 106-112 *(e.g.,* the remainder of the core wire 120). For example, the portions 106-112 may have a width or diameter of about 0.011 inches (about 0.3 mm), the distal segment 136 and the distal tip 116 may have a width or diameter of about 0.007 inches (about 0.2 mm), and the narrow segment 130 may have a width or diameter of about 0.004 inches (about 0.1 mm). The proximal taper 132 may have a longer length and a more gradual slope than the distal taper 134, allowing the proximal taper 132 to withstand greater forces during insertion and prevent stress concentrations/weak points compromising the strength/performance of the stylet 100. For example, the proximal taper 132 may be at least about twice the length of the distal taper 134 and less than about six times the length of the distal taper 134 (*e*.*g*., at least three, four, or five times the length of the distal taper 134). Furthermore, a length between the proximal end of the narrow segment 130' and the distal tip of the stylet 100' may be less than or equal to about 1.6 inches, *e.g*., about 0.6-1.4 inches (less than or equal to about 4 cm, *e.g.,* about 1.5-3.5 cm) to provide favorable deflection mechanics.

A conductive coil 160 may be disposed over and around the distal segment 136 of the core wire 120 to provide passive sensor coil capabilities for navigation. As illustrated in FIG. 4 (and omitted in FIGS. 2-3 for clarity), a first lead 162 may integrally connect to a distal end of the conductive coil 160, wrap around the core wire 120 (*e.g.,* 5 times), and electrically connect to a circuit board (not shown) inside the housing member 102. Similarly, a second lead 164 may integrally connect to a proximal end of the conductive coil 160, wrap around the core wire 120 (*e.g.,* 5 times), and electrically connect to the circuit board inside the housing member 102. The conductive coil 160 and leads 162, 164 may each be a conductive copper wire. In use, the conductive coil 160 may pick up electromagnetic signals generated and transmitted by one or more drive coils (not shown) external of the body. The resultant signal of the conductive coil 160 may be transmitted via at least one of the leads 162, 164 to the circuit board in the housing member 102. The circuit board may transmit the signal to a computer (not shown) to display a location of the fifth portion 114. Further discussion of the structure and function of the conductive coil 160 and tip navigation systems may be found in U.S. Patent No. 10,098,567.

The tubular body 140 may be disposed over the core wire 120 and enclose the conductive coil 160 and leads 162, 164. The tubular body 140 may secure the leads 162, 164 and prevent the leads 162, 164 from coming loose and/or contacting fluid during insertion. The tubular body 140 may be made of a soft and flexible polymer (e.g., polyimide or nylon), not having a significant function or impact on the bend profile of the stylet 100. The tubular body 140 may include a lubricious coating or outer layer to decrease frictional forces between the catheter 150 to facilitate removal of the stylet 100. For example, the tubular body 140 may include a polytetrafluorethylene (PTFE) outer layer or a hydrophilic coating. The tubular body 140 may have a variation in frictional properties along its length, such that a distal portion of the tubular body 140 is more lubricous that a proximal portion of the tubular body 140. In some embodiments, the frictional variation may be provided by increased surface texture, with the distal portion of the tubular body being smoother than the proximal portion of the tubular body, and/or the proximal portion being more textured, roughened, or ribbed. The distal end of the tubular body 140 may be bonded to the core wire 120 distal of the conductive coil 160. For example, the distal end of the tubular body 140 may be bonded and/or sealed with a Cyanoacrylate adhesive to the core wire 120 to prevent fluid from entering the tubular body 140. In some embodiments, the tubular body 140 may include markings for the user to be able to visualize when the stylet 100 is sliding (intentionally and unintentionally) relative to the catheter 150.

The distal tip 116 formed by the core wire 120 may extend distally from the distal end of tubular body 140, and the distal tip 116 may be atraumatic (e.g., rounded and/or tapered edges) to reduce trauma on the vascular tissue. The distal tip 116 may be exposed to ensure adequate conduction of Electrocardiogram (ECG) signals that assist in determining proper positioning in the SVC. The ECG signals may be further conducted along the length of core wire 120 to the circuit board (not shown) inside the housing member 102. The ECG conductivity and position determination are further discussed in U.S. Pat. No. 10,321,890.

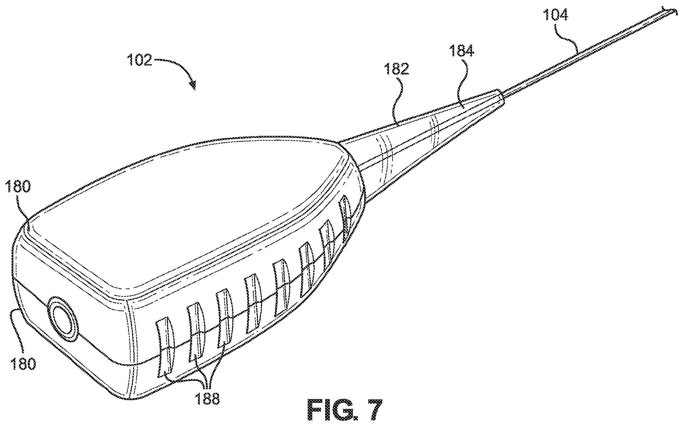

FIG. 7 illustrates an isometric view of the housing member 102. As illustrated, the housing member 102 may include first and second housing portions 180 and a nose member 182. The first and second housing portions 180 may be assembled to house electronic components (e.g., the circuit board) connected to the conductive coil 160 via the leads 162, 164. For example, the first and second portions 180 may have corresponding pins and holes configured to press together to form the housing member 102. Each of the first and second housing portions 180 may include grooves 188 on side surfaces that join to provide enhanced grip of the user. Each of the first and second housing portions 180 may also include protruding walls to be received in a neck or groove of the nose member 182 to secure the nose member 182 to the housing member 102 when assembled. The nose member 182 may define a sleeve having a lumen therethrough to receive the elongated body 104 into the housing member 102. The nose member 182 may have a tapered distal portion 184 extending distally from the first and second housing portions 180 and a proximal portion received between the first and second housing portions 180. The nose member 182 may be made of an elastomeric material (e.g., silicone rubber) to provide strain relief for the attachment of the elongated body 104 to the housing 102.

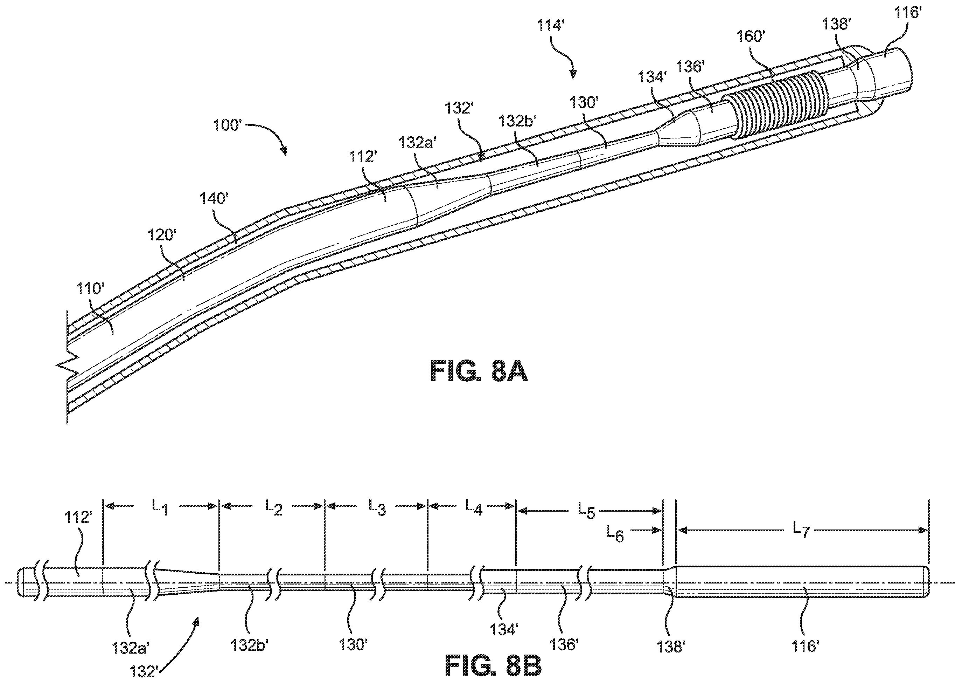

FIGS. 8A-B illustrate a stylet 100' according to a second embodiment of the present invention. The stylet 100' may have similar features as discussed above with reference to the stylet 100, including the stiffness profile and indicated with corresponding reference numbers, including the portions 110-116, 130, 136 (with corresponding portions 110'-116', 130', 136' shown), and a tubular body 140'. Thus, as further discussed herein, the stylet 100' may have the pre-formed first and/or second bends α, β; however, in some embodiments, the stylet 100' may have a pre-formed substantially straight configuration (omitting the first and second bends α, β. In some embodiments, the stylet 100' may have a proximal taper 132' with a first tapered portion 132a' and a second tapered portion 132b' that are connected but have different taper angles. The first tapered portion 132a' may be proximal of the second tapered portion 132b' and transition from the fourth portion 112'. The first tapered portion 132a' may be tapered at a larger angle relative to the longitudinal axis than the second tapered portion 132b'. With the second tapered portion 132b' having a more gradual angle at the proximal end of the narrow segment 130', some of the yielding that occurs in the narrow segment 130' may be transmitted into the larger angle of the first tapered portion 132a'. This reduces the abruptness of the transition. The larger angle of the first taper portion 132a' may also provide a slightly steeper transition to reduce the overall length of the proximal taper 132'. The fifth portion 114' may include the distal segment 136' receiving the conductive coil 160' positioned distal of a first distal taper 134', and a second distal taper 138' between the distal segment 136' and the distal tip 116'. Thus, the distal tip 116' may have substantially the same width or diameter as at least one of the proximal portions 106-112 (as illustrated in the embodiment of FIG. 2). The narrow segment 130' may have a constant width or diameter. The distal segment 136' may have a constant width or diameter greater than the narrow segment 130' and less than the distal tip 116'. For example, the distal segment 136' may have a width or diameter of about 0.009 inch (about 0.22 mm) and the distal tip 116' may be larger and unground, having a width or diameter of about 0.011 inch (about 0.3 mm) increasing the surface area for ECG conductivity,

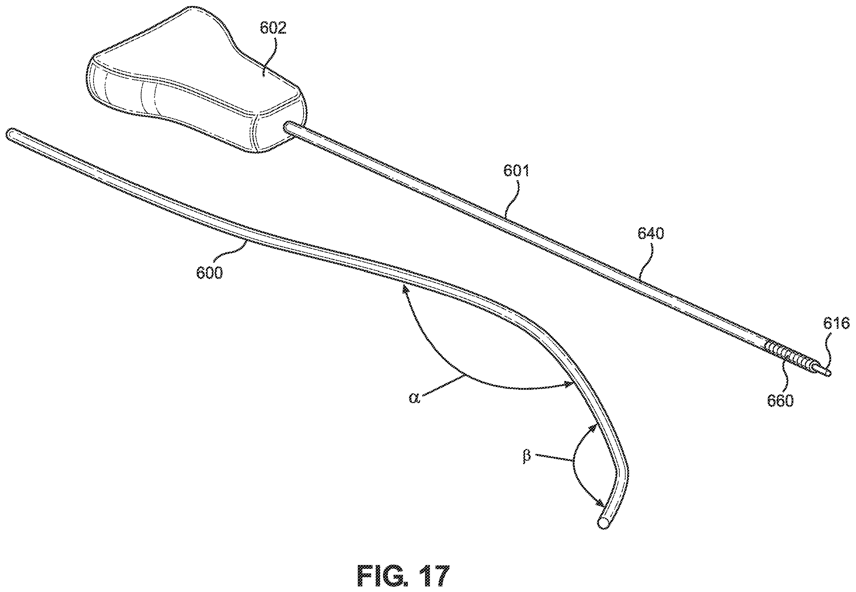

As further illustrated in FIG. 8B, the first taper portion 132a' may have a first length (Li), the second taper portion 132b' may have a second length (L2), the narrow segment 130' may have a third length (L3), the first distal taper 134' may have a fourth length (L4), the distal segment 136' may have a fifth length (L5), the second distal taper 138' may have a sixth length (L6), and the distal tip 116' may have a seventh length (L7). The proximal taper 132' (including the first and second taper portions 132a', 132b') may be more gradual and have a length (Li+L2) that may be longer than that of the first and/or second distal tapers 134', 138' (L4, L6) and/or combined lengths thereof, allowing the proximal taper 132' to withstand greater forces during insertion and prevent stress concentrations/weak points compromising the strength/performance of the stylet 100'. The lengths of the first and second distal tapers 134', 138' may be reduced to allow the width of the stylet 100' to increase distally over short distances. For example, the proximal taper 132' may be at least about twice the length of at least one of the first and second distal tapers 134', 138' (e.g., both) and less than about six times the length of at least one of the first and second distal tapers 134', 138' (e.g., only the first distal taper 134'). In one illustrative example, the first length (Li) may be about 0.1-0.3 inches, e.g., about 0.2 inches (about 2.5-7.5 mm, e.g., about 5 mm), the second length (L2) may be about 0.1-0.3 inches, e.g., about 0.2 inches (about 2.5- 7.5 mm, e.g., about 5 mm), and the third length (L3) may be about 0.1-0.3 inches, e.g., about 0.2 inches (about 2.5-7.5 mm, e.g., about 5 mm). The fourth length (L4) may be about 0.1-0.2 inches, e.g., about 0.14 inches (about 2.5-5 mm, e.g., about 3.5 mm), fifth length (L5) may be about 0.5-0.75 inches, e.g., about 0.635 inches (about 12.5-20 mm, e.g., about 16 mm), the sixth length (L6) may be about 0.005-0.015 inches, e.g., about 0.010 inches (about 0.125-0.4 mm, e.g., about 0.25 mm), and seventh length (L7) may be about 0.05-0.15 inches, e.g., about 0.1 inches (about 1.25-4 mm, e.g., about 2.5 mm). Thus, a length between the proximal end of the narrow segment 130' and the distal end of the stylet 100' may be less than or equal to about 1.6 inches, e.g. about 0.6-1.4 inches (less than or equal to about 4 cm, e.g., about 1.5- 3.5 cm) to provide favorable deflection mechanics. The stylet 100' may be made of a nickel titanium alloy (Nitinolln or stainless steel and have a polytetrafluoroethylene (PTFE) or hydrophobic coating. The stylet 100' may be without a navigation device (omitting the conductive coil 160, leads 162, 164, and tubular body 140), and used as a stiffening wire alongside a separate navigation stylet (e.g., as illustrated in FIGS. 17-19) or by itself (e.g., assembled with the PICC without another stylet).

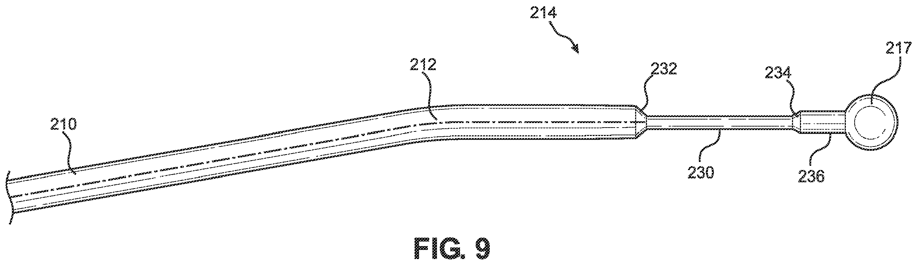

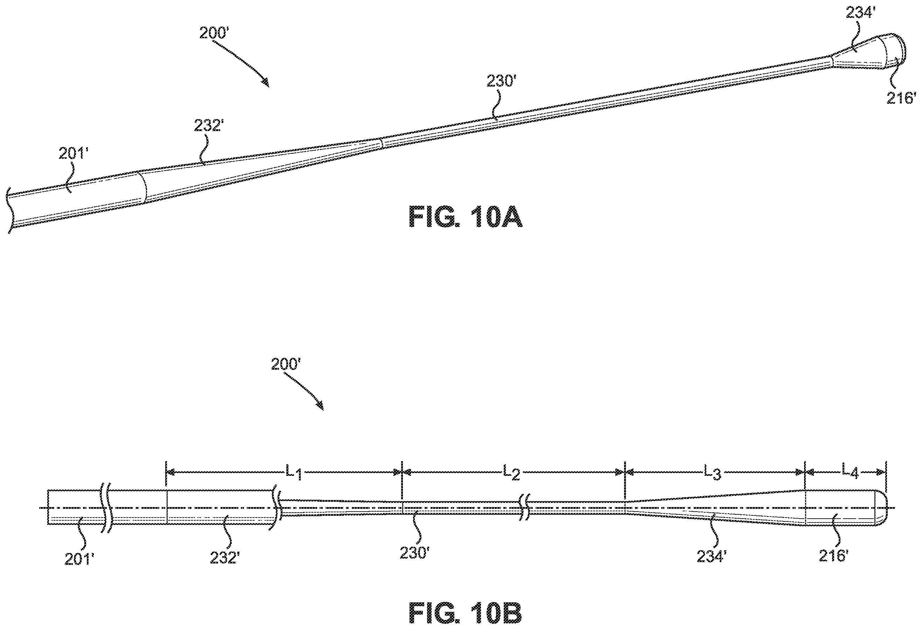

FIG. 9 illustrates a stylet 200 according to a third embodiment of the present invention. The stylet 200 may have similar features as discussed above with regard to at least one of the stylets 100, 100', including. the stiffness profile with regard to the stylet 100 and indicated with corresponding reference numbers, including the portions 110-114, 130, 1.36 (with corresponding portions 210-214, 230, 236 shown), proximal and distal tapers 132, 134 (with corresponding tapers 232, 234 shown). As further discussed herein, the stylet 200 may have the pre-formed first and/or second bends α, β; however, in some embodiments, the stylet 200 may have a pre-formed substantially straight configuration (omitting the first and second bends α, β). The stylet 200 may have a distal tip 21.6 including an atraumatic shape, such as a ball weld 217 or a larger diameter with a rounded edge (e.g., as illustrated in FIG. 10A-B). The stylet 200 may be without a navigation device (omitting the conductive coil 160, leads 162, 164, and tubular body 140), and used as a stiffening wire alongside a separate navigation stylet (e.g., as illustrated in FIGS. 17-19) or by itself (e.g., assembled with the PICC without another stylet).

FIGS. 10A-B illustrate a stylet 200' according to a fourth embodiment of the present invention. The stylet 200' may have similar features as discussed above with regard to at least one of the stylets 100, 100', 200 . As illustrated, the stylet 200' may include a proximal portion 201' extending from a proximal end (not shown), a proximal taper 232', a narrow segment 230', a distal taper 234', and a distal tip 216' extending to a distal end, in that longitudinal order. As further illustrated, the stylet 200' may have a pre-formed substantially straight configuration (omitting the first and second bends α, β); however, in some embodiments, the style 200' may have the pre-formed first and/or second bends α, β, as further discussed herein. The proximal portion 201' may have a first width (or diameter), and the proximal taper 232' may reduce the width of the stylet 200' to a second width (or diameter) less than the first width at the narrow segment 230'. The distal taper 234' may extend from the narrow segment 230' and increase the width of the stylet 200' to a third width (or diameter) at the distal tip 216', where the first and third widths may be substantially the same. The second width of the narrow segment 230' may be less than the first and second widths to provide a floppy portion to allow deflection of the distal portion of the stylet 200', for example, down the RBCV (as further discussed herein). Furthermore, the third width of the distal tip 216' may be large enough to ensure that the distal tip 216' is atraumatic and/or the distal tip 216' may include a substantially cylindrical portion with a rounded and/or tapered circumferential edge.

As further illustrated in FIG. 10B, the proximal taper 232' may have a first length (Li), the narrow segment 230' may have a second length (L2), the distal taper 234' may have a third length (L3), and the distal tip 216' may have a fourth length (L4). The proximal taper 232' may be more gradual and the first length (Li) may be longer than the third length (L3), allowing the proximal taper 232' to withstand greater forces during insertion and prevent stress concentrations/weak points compromising the strength/performance of the stylet 200'. The third length (L3) of the distal taper 234' may be reduced to allow the width of the stylet 200' to increase from the narrow segment 230' to the distal tip 216' over a short distance. For example, the proximal taper 232' may be at least about twice the length of the distal taper 234' and less than about six times the length of the distal taper 234'. In one illustrative example, the first length (Li) may be about 0.2-0.6 inches, e.g., about 0.4 inches (about 5-15 mm e.g, about 10 mm), the second length (L2) may be about 0.6-1.0 inches, e.g., about 0.8 inch (about 15-25 mm, e.g., about 20 mm), the third length (L3) may be about 0.04-0.2 inches, e.g., about 0.08 inches (about 1 to 5 mm, e.g., about 2 mm), and the fourth length (1.4) may be about 0.02-0.06 inches, e.g., about 0.04 inches (about 0.5-1.5 mm, e.g., about 1 mm). Thus, a fifth length (1,2-1-L3-i-L4) between the proximal end of the narrow segment 230' and the distal tip of the stylet 200' may be less than or equal to about 1.6 inches, e.g., about 0.6-1.4 inches (less than or equal to about 40 mm, e.g.. about 15-35 mm) to provide favorable deflection mechanics. The stylet 200' may be made of a nickel titanium alloy or stainless steel and have a polytetrafluoroethylene (PTFE) or hydrophobic coating. The stylet 200' may be without a navigation device (omitting the conductive coil 160, leads 162, 164, and tubular body 140), and used as a stiffening wire alongside a separate navigation stylet (e.g., as illustrated in FIGS. 17-19) or by itself (e.g., assembled with the PICC without another stylet).

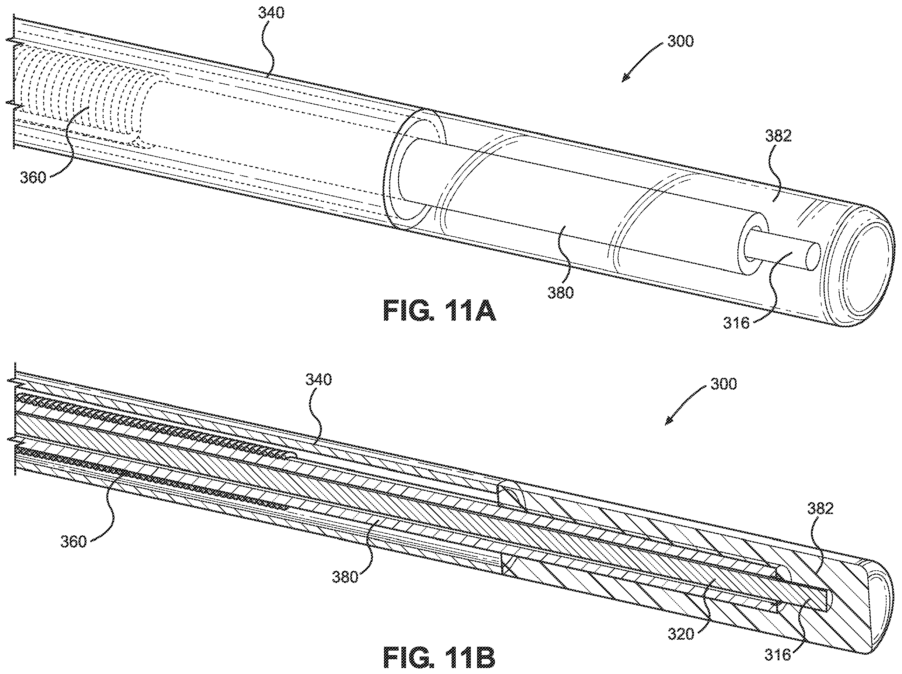

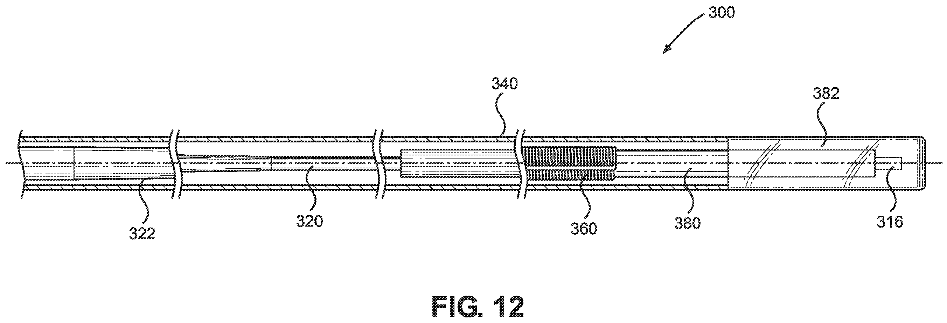

FIGS. 11A-12 illustrate a stylet 300 according to a fifth embodiment of the present invention. The stylet 300 may include a core wire 320, a tubular body 340, a conductive coil 360, and a magnetic hypotube 380. As illustrated, the core wire 320 may be received inside of the magnetic hypotube 380, and the core wire 320 and magnetic hypotube 380 may be received in the tubular body 340. The stylet 300 may have similar features as discussed above with regard to at least one of the stylets 100, 100', 200, including the body and tip curve profile, sizes, and stiffness profile with regard to the stylet 100, the portions 110-116, 130, and the first and second bends α, β. The core wire 320 may be made of a nickel titanium alloy, providing the additional advantage of being a kink-resistant alloy, but is inherently non-magnetic. Therefore, in order to provide the desired properties of the present application (e.g., navigation electronic stylet with curve profile and appropriate grind profile), the magnetic hypotube 380 may be provided over a distal portion of the core wire 320. The magnetic hypotube 380 may be short and made out of a magnetic permeable material, such as stainless steel or other similar materials. The core wire 320 may include a taper 322 disposed proximally of the magnetic hypotube 380, and the hypotube 380 may be disposed proximally of a distal tip 326 of the core wire 320. The conductive coil 360 may be wound over the hypotube 380 to provide passive sensor coil capabilities for navigation, as discussed herein.

The core wire 320 and the magnetic hypotube 380 may extend distally out of the tubular body 340 and be at least partially covered with a conductive epoxy 382 at a portion of the stylet 300 distal of the tubular body 340. In some embodiments, the conductive epoxy 382 may secure the core wire 320, tubular body 340, and magnetic hypotube 380 together and be configured to conduct the ECG signals to the tip portion 316. However, in some embodiments, a second epoxy (not shown) may join the core wire 320 and the magnetic hypotube 380, while the conductive epoxy 382 covers the tip portion 316 to conduct the ECG signals. Additionally or alternatively, the core wire 320 and hypotube 380 may be welded together with enough exposed metal at the tip portion 316 to conduct the ECG signals. Additionally or alternatively, a conductive hypotube (not shown) may be provided to conduct the ECG signals.

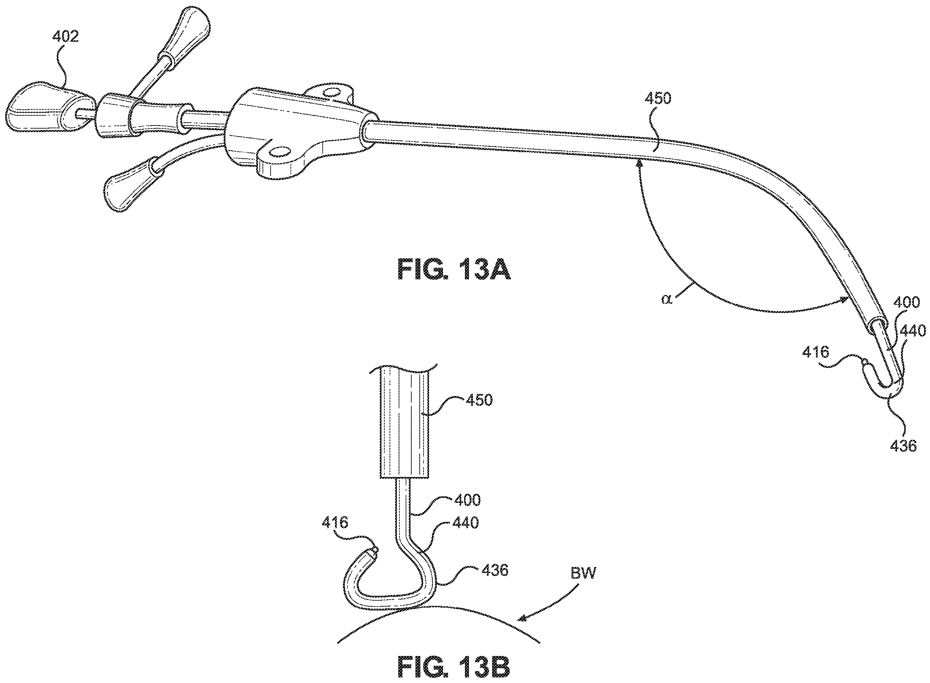

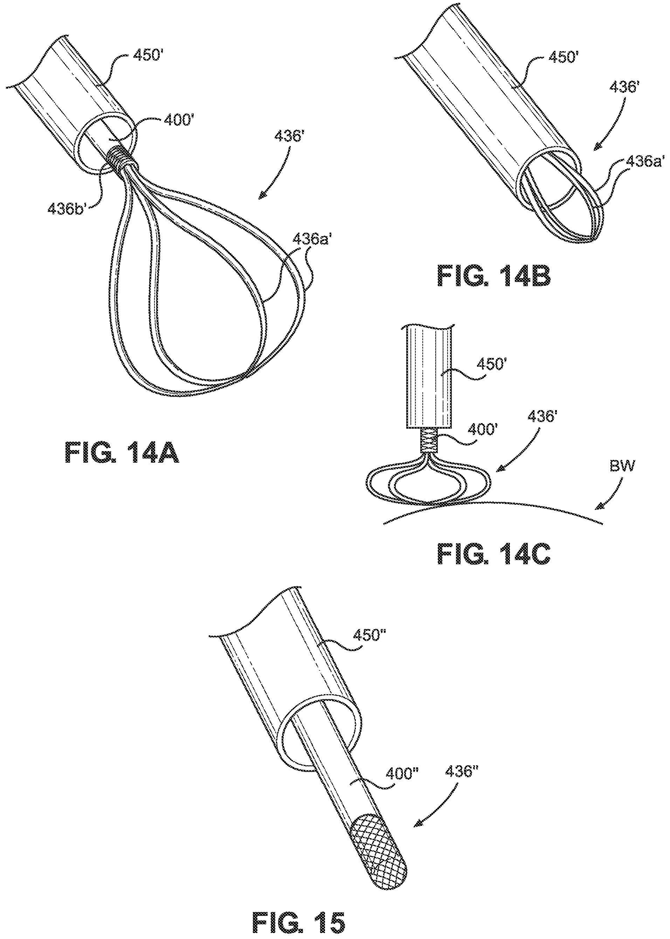

FIGS. 13A-B illustrate a stylet 400 and a catheter 450 according to a sixth embodiment of the present invention. The stylet 400 may have similar features as discussed above with regard to at least one of the stylets 100, 100', 200, 300, including a core wire, a tubular body 440, and the first and second bends α, β. The stylet 400 may further include an atraumatic distal segment 436 that reduces trauma to a back wall (BW) at the confluence (C) and facilitates deflection of the distal segment 436 down/off the back wall (BW) toward the RBVC, compared to a stylet/PICC assembly with a leading tip comprised externally of a P1CC only (e.g., without an atraumatic distal segment). The distal segment 436 may be soft and/or substantially compressible along the longitudinal axis of the stylet 400 and extend distally of the distal end of the catheter 450 to contact the vein first. For example, as illustrated in FIGS. 13A-B, the distal segment 436 may have a .1-shape that is configured to resiliently deflect when contacting the back wall (BW) and reduce "tenting" compared to a more pointed distal portion. The core wire may extend through the distal segment 436, such that a distal end 416 of the core wire extends out of the tubular body 440 in a proximal direction. The distal end 416 may be exposed to allow for conduction of ECG signals, as further discussed herein. In another embodiment, as illustrated in FIGS. 14A-C, a stylet 400' received in a catheter 450' may include a distal segment 436' including a plurality of flexible members 436a' (e.g., wires) having a curved or looped configuration forming a basket. The flexible members 436a' may extend distal from a tubular portion 436b' and be joined at a distal tip of the stylet 400' and/or be looped back proximally to the tubular portion 436b'. The flexible members 436a' may be resiliently expandable (as illustrated in FIG. 14A) and be collapsed when pulled back into the catheter 450' (as illustrated in FIG. 14B). The flexible members 436a' may be conductive of ECG signals for navigation purposes, as discussed herein. Similar to the embodiment of FIG. 13A-B, the distal segment 436' may resiliently deflect when contacting the back wall (BW) (as illustrated in FIG. 14C) at the confluence (C) and reduce "tenting". In yet another embodiment, a stylet 400" received in a catheter 4.50" may include a distal segment 436" of a soft mesh of a plurality of members. The distal segment 436" may resiliently deflect when contacting the back wall (BW) at the confluence (C) and reduce "tenting". The distal segment 436" may further be conductive of ECG signals, as discussed herein. The function of the distal segment 436, 436', 436" may supplement or replace the functionality of the first bend α. and/or second bend β, thus in some embodiments, the first bend α and/or second bend β may be omitted, such that the stylet 400 may have a pre-formed straight configuration.

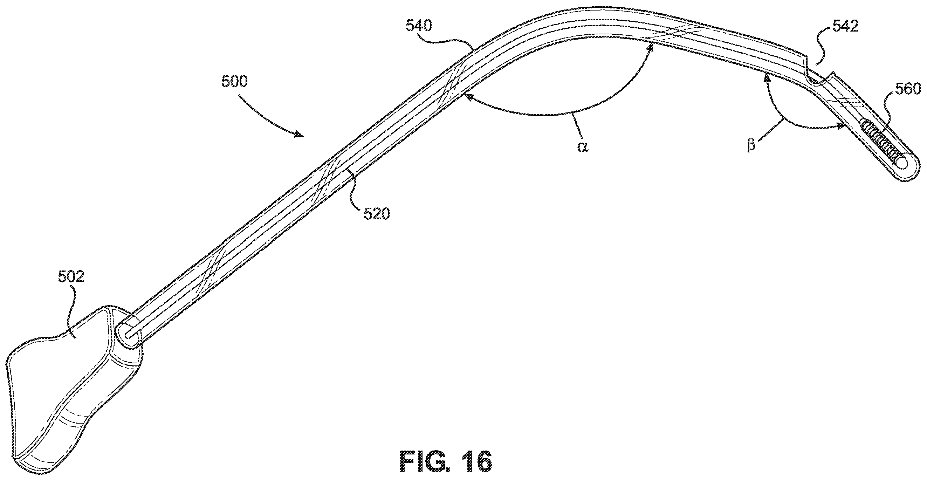

FIG. 16 illustrates a stylet 500 according to a seventh embodiment of the present invention. The stylet 500 may have similar features as discussed above with regard to at least one of the stylets 100, 100', 200, 300, 400, including a core wire 520, a tubular body 540, a housing member 502, and first and second bends α, β. As illustrated, the distal end of the core wire 520 may be enclosed by the tubular body 540 reducing trauma and providing a lubricous contact with the back wall (BW) at the confluence (C). To provide the ECG signal conduction, the tubular body 540 may include an opening 542 through a side wall of the tubular body 540 between the proximal and distal ends of the tubular body 540. The opening 542 may allow for ECG signals from the body to contact the core wire 520. For example, the opening 542 may be distal of the first and/or second bends α, β (e.g., at the second bend β, as illustrated) in order to detect the location of the distal tip of the stylet 500. Thus, the core wire 520 may be configured to conduct the ECG signal for navigation, while avoiding any trauma caused by an exposed distal tip of the core wire 520, if it were sharp.

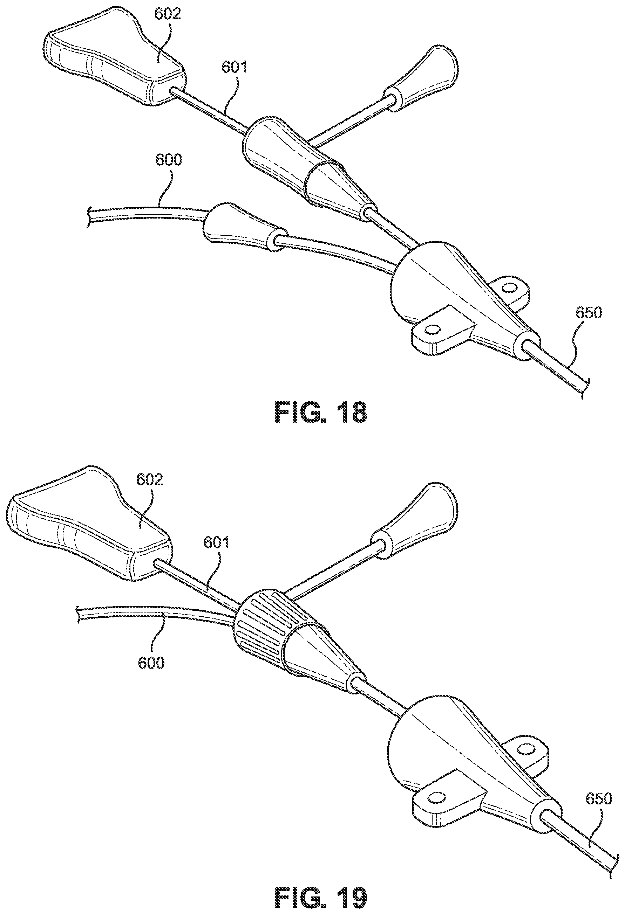

FIGS. 17-19 illustrate a pair of stylets 600, 601 according to an eighth embodiment of the present invention. The first style 600 may be configured to be conductive of an ECG signal for navigation, and the second stylet 601 may be configured to shape a catheter 650 to facilitate threading into the SVC, as further discussed herein with regard to at least one of the stylets 100, 100', 200, 300, 400, 500. The first stylet 600 may include a housing 602, a tubular body 640, a core wire disposed within the tubular body 640 and having a distal tip 616 to conduct the ECG signal, and conductive coil 660 around the core wire in the tubular body 640, as further discussed herein with regard to at least one of the stvlets 100, 100', 200, 300, 400, 500. The second stylet 601 may be a non-conductive elongated member or wire pre-formed with the first and second bends α, β to facilitate threading the catheter 650 past the confluence (C). As illustrated in the first assembly of FIG. 18, the first and second stvlets 600, 601 may be inserted in separate lumens of the catheter 650. As further illustrated in the second assembly of FIG. 19, the first and second stylets 600, 601 may be inserted into the same lumen of the catheter 6.50. In either assembly, separating the functions (e.g., ECG navigation and threadability of the catheter) into the first and second stylets 600, 601 may facilitate manufacturing, for example, by allowing different materials with optimized mechanical and/or electrical properties to perform each function.

## Claims

1. A stylet (100, 100') comprising:
a core wire (120, 120') having a pre-formed shape, the core wire including:
a first portion (106) being substantially straight;
a second portion (108) extending distally from the first portion (106) and having a first pre-formed bend of a first angle;
a third portion (110, 110') extending distally from the second portion (108) and being substantially straight;
a fourth portion (112, 112') extending distally from the third portion (110, 110') and having a second pre-formed bend of a second angle;
a fifth portion (114, 114') extending distally from the fourth portion (112, 112') and to a distal tip (116, 116'), the fifth portion being substantially straight;
the first angle defined between a longitudinal axis of the first portion and a longitudinal axis of the third portion, the second angle defined between the longitudinal axis of the third portion and a longitudinal axis of the fifth portion, the second pre-formed bend being distal of the first pre-formed bend, and the first angle being greater than the second angle; and
a narrow segment (130, 130') being distal of the second pre-formed bend and having a reduced width or diameter that increases deflection of the distal tip,
wherein the first pre-formed bend and the second pre-formed bend are in the same plane and the same direction.

2. The stylet of claim 1, wherein the first angle is about 15-90° with respect to a longitudinal axis of the stylet, and an arc length of the first pre-formed bend is about 7.62 - 17.78cm (3-7 inches).

3. The stylet of claim 1 or claim 2, wherein the second angle is about 8-15° with respect to a longitudinal axis of the stylet, and an arc length of the second pre-formed bend is about 0.508-1.27cm (0.2-0.5 inches).

4. The stylet of at least one of the preceding claims, wherein the first pre-formed bend and the second pre-formed bend are entirely within 25.4 cm ( 10 inches) from an end of the distal tip of the core wire.

5. The stylet of at least one of the preceding claims, wherein the first angle is about 60° with respect to a longitudinal axis of the stylet, and the second angle is about 10° with respect to the longitudinal axis of the stylet.

6. The stylet of at least one of the preceding claims, wherein the first pre-formed bend is longer than the second pre-formed bend.

7. The stylet of claim 6, wherein the first pre-formed bend has an arc length of at least 10.16cm (4 inches) longer than the second pre-formed bend.

8. The stylet of at least one of the preceding claims, wherein the narrow segment (130, 130') includes a rounded and/or flattened cross-section.

9. The stylet of at least one of the preceding claims, wherein the core wire (120, 120') comprises a distal segment (136, 136') between the narrow segment (130, 130') and the distal tip (116, 116'), the distal segment having a width or diameter greater than the narrow segment.

10. The stylet of claim 9, wherein the distal tip has a width or diameter greater than the distal segment.

11. The stylet of at least one of the preceding claims, wherein the core wire (120, 120') includes a proximal taper (132, 132') proximal of the narrow segment (130, 130') and distal taper (134, 134') distal of the narrow segment.

12. The stylet of claim 11, wherein the proximal taper is longer than the distal taper.

13. The stylet of claim 11 or claim 12, wherein the proximal taper includes a first tapered portion (132a') and a second tapered portion (132b'), the first tapered portion being tapered at a greater angle than the second tapered portion.

14. The stylet of at least one of the preceding claims, further comprising:
a tubular body (140), 140') disposed over the core wire; and optionally
a navigation device (160, 160') disposed over the core wire and within the tubular body.

15. The stylet of claim 14, wherein the core wire (120, 120') is conductive of an electrocardiogram, ECG, signal, and the distal tip (116, 116') of the core wire extends distally of the tubular body (140, 140') or the tubular body has an opening in a sidewall to allow fluid contact with the core wire.

## Patentansprüche

1. Mandrin (100, 100'), umfassend:
einen Kerndraht (120, 120') mit einer vorgeformten Form, wobei der Kerndraht beinhaltet:
einen ersten Abschnitt (106), der im Wesentlichen gerade ist;
einen zweiten Abschnitt (108), der sich distal von dem ersten Abschnitt (106) erstreckt und eine erste vorgeformte Biegung mit einem ersten Winkel aufweist;
einen dritten Abschnitt (110, 110'), der sich distal von dem zweiten Abschnitt (108) erstreckt und im Wesentlichen gerade ist;
einen vierten Abschnitt (112, 112'), der sich distal von dem dritten Abschnitt (110, 110') erstreckt und eine zweite vorgeformte Biegung mit einem zweiten Winkel aufweist;
einen fünften Abschnitt (114, 114'), der sich distal von dem vierten Abschnitt (112, 112') und zu einer distalen Spitze (116, 116') hin erstreckt, wobei der fünfte Abschnitt im Wesentlichen gerade ist;
wobei der erste Winkel zwischen einer Längsachse des ersten Abschnitts und einer Längsachse des dritten Abschnitts definiert ist, der zweite Winkel zwischen der Längsachse des dritten Abschnitts und einer Längsachse des fünften Abschnitts definiert ist, die zweite vorgeformte Biegung distal von der ersten vorgeformten Biegung ist und der erste Winkel größer ist als der zweite Winkel; und
ein schmales Segment (130, 130') sich distal von der zweiten vorgeformten Biegung befindet und eine reduzierte Breite oder einen reduzierten Durchmesser aufweist, welches die Auslenkung der distalen Spitze erhöht,
wobei sich die erste vorgeformte Biegung und die zweite vorgeformte Biegung in der gleichen Ebene und der gleichen Richtung befinden.

2. Mandrin nach Anspruch 1, wobei der erste Winkel etwa 15-90° in Bezug auf eine Längsachse des Mandrins beträgt und eine Bogenlänge der ersten vorgeformten Biegung etwa 7,62-17,78 cm (3-7 Zoll) beträgt.

3. Mandrin nach Anspruch 1 oder 2, wobei der zweite Winkel etwa 8-15° in Bezug auf eine Längsachse des Mandrins beträgt und eine Bogenlänge der zweiten vorgeformten Biegung etwa 0,508-1,27 cm (0,2-0,5 Zoll) beträgt.

4. Mandrin nach mindestens einem der vorhergehenden Ansprüche, wobei die erste vorgeformte Biegung und die zweite vorgeformte Biegung vollständig innerhalb von 25,4 cm (10 Zoll) von einem Ende der distalen Spitze des Kerndrahts entfernt liegen.

5. Mandrin nach mindestens einem der vorhergehenden Ansprüche, wobei der erste Winkel in Bezug auf eine Längsachse des Mandrins etwa 60° beträgt und der zweite Winkel in Bezug auf die Längsachse des Mandrins etwa 10° beträgt.

6. Mandrin nach mindestens einem der vorhergehenden Ansprüche, wobei die erste vorgeformte Biegung länger ist als die zweite vorgeformte Biegung.

7. Mandrin nach Anspruch 6, wobei die erste vorgeformte Biegung eine Bogenlänge aufweist, die mindestens 10,16 cm (4 Zoll) länger ist als die zweite vorgeformte Biegung.

8. Mandrin nach mindestens einem der vorhergehenden Ansprüche, wobei das schmale Segment (130, 130') einen abgerundeten und/oder abgeflachten Querschnitt beinhaltet.

9. Mandrin nach mindestens einem der vorhergehenden Ansprüche, wobei der Kerndraht (120, 120') ein distales Segment (136, 136') zwischen dem schmalen Segment (130, 130') und der distalen Spitze (116, 116') umfasst, wobei das distale Segment eine größere Breite oder einen größeren Durchmesser aufweist als das schmale Segment.

10. Mandrin nach Anspruch 9, wobei die distale Spitze eine größere Breite oder einen größeren Durchmesser aufweist als das distale Segment.

11. Mandrin nach mindestens einem der vorhergehenden Ansprüche, wobei der Kerndraht (120, 120') eine proximale Verjüngung (132, 132') proximal des schmalen Segments (130, 130') und eine distale Verjüngung (134, 134') distal des schmalen Segments beinhaltet.

12. Mandrin nach Anspruch 11, wobei die proximale Verjüngung länger ist als die distale Verjüngung.

13. Mandrin nach Anspruch 11 oder 12, wobei die proximale Verjüngung einen ersten sich verjüngenden Abschnitt (132a') und einen zweiten sich verjüngenden Abschnitt (132b') beinhaltet, wobei der erste sich verjüngende Abschnitt in einem größeren Winkel verjüngt ist als der zweite sich verjüngende Abschnitt.

14. Mandrin nach mindestens einem der vorhergehenden Ansprüche, ferner umfassend:
einen röhrenförmigen Körper (140, 140'), der über dem Kerndraht angeordnet ist; und optional
eine Navigationsvorrichtung (160, 160'), die über dem Kerndraht und innerhalb des rohrförmigen Körpers angeordnet ist.

15. Mandrin nach Anspruch 14, wobei der Kerndraht (120, 120') für ein Elektrokardiogramm(EKG)-Signal leitfähig ist und sich die distale Spitze (116, 116') des Kerndrahts distal von dem röhrenförmigen Körper (140, 140') erstreckt oder der röhrenförmige Körper eine Öffnung in einer Seitenwand aufweist, um Fluidkontakt mit dem Kerndraht zu ermöglichen.

## Revendications

1. Stylet (100, 100') comprenant :
un fil métallique central (120, 120') ayant une forme préformée, le fil métallique central comportant :
une première partie (106) étant sensiblement droite ;
une deuxième partie (108) s'étendant distalement à partir de la première partie (106) et présentant un premier coude préformé d'un premier angle ;
une troisième partie (110, 110') s'étendant distalement à partir de la deuxième partie (108) et étant sensiblement droite ;
une quatrième partie (112, 112') s'étendant distalement à partir de la troisième partie (110, 110') et présentant un second coude préformé d'un second angle ;
une cinquième partie (114, 114') s'étendant distalement à partir de la quatrième partie (112, 112') et jusqu'à une pointe distale (116, 116'), la cinquième partie étant sensiblement droite ;
le premier angle étant défini entre un axe longitudinal de la première partie et un axe longitudinal de la troisième partie, le second angle étant défini entre l'axe longitudinal de la troisième partie et un axe longitudinal de la cinquième partie, le second coude préformé étant distal par rapport au premier coude préformé, et le premier angle étant supérieur au second angle ; et
un segment étroit (130, 130') étant distal par rapport au second coude préformé et ayant une largeur réduite ou un diamètre réduit qui augmente la déflexion de la pointe distale,
dans lequel le premier coude préformé et le second coude préformé sont dans le même plan et dans la même direction.

2. Stylet selon la revendication 1, dans lequel le premier angle est d'environ 15 à 90° par rapport à un axe longitudinal du stylet, et une longueur d'arc du premier coude préformé est d'environ 7,62 à 17,78 cm (3 à 7 pouces).

3. Stylet selon la revendication 1 ou la revendication 2, dans lequel le second angle est d'environ 8 à 15° par rapport à un axe longitudinal du stylet, et une longueur d'arc du second coude préformé est d'environ 0,508 à 1,27 cm (0,2 à 0,5 pouce).

4. Stylet selon au moins l'une des revendications précédentes, dans lequel le premier coude préformé et le second coude préformé se trouvent entièrement à moins de 25,4 cm (10 pouces) d'une extrémité de la pointe distale du fil métallique central.

5. Stylet selon au moins l'une des revendications précédentes, dans lequel le premier angle est d'environ 60° par rapport à un axe longitudinal du stylet, et le second angle est d'environ 10° par rapport à l'axe longitudinal du stylet.

6. Stylet selon au moins l'une des revendications précédentes, dans lequel le premier coude préformé est plus long que le second coude préformé.

7. Stylet selon la revendication 6, dans lequel le premier coude préformé a une longueur d'arc d'au moins 10,16 cm (4 pouces) plus longue que le second coude préformé.

8. Stylet selon au moins l'une des revendications précédentes, dans lequel le segment étroit (130, 130') comporte une section transversale arrondie et/ou aplatie.

9. Stylet selon au moins l'une des revendications précédentes, dans lequel le fil métallique central (120, 120') comprend un segment distal (136, 136') entre le segment étroit (130, 130') et la pointe distale (116, 116'), le segment distal ayant une largeur ou un diamètre supérieurs au segment étroit.

10. Stylet selon la revendication 9, dans lequel la pointe distale a une largeur ou un diamètre supérieurs au segment distal.

11. Stylet selon au moins l'une des revendications précédentes, dans lequel le fil métallique central (120, 120') comporte un cône proximal (132, 132') proximal par rapport au segment étroit (130, 130') et un cône distal (134, 134') distal par rapport au segment étroit.

12. Stylet selon la revendication 11, dans lequel le cône proximal est plus long que le cône distal.

13. Stylet selon la revendication 11 ou la revendication 12, dans lequel le cône proximal comporte une première partie effilée (132a') et une seconde partie effilée (132 b'), la première partie effilée étant effilée selon un angle plus grand que la seconde partie effilée.

14. Stylet selon au moins l'une des revendications précédentes, comprenant en outre :
un corps tubulaire (140, 140') disposé sur le fil central ; et éventuellement
un dispositif de navigation (160, 160') disposé sur le fil métallique central et à l'intérieur du corps tubulaire.

15. Stylet selon la revendication 14, dans lequel le fil métallique central (120, 120') est conducteur d'un signal d'électrocardiogramme, d'ECG, et la pointe distale (116, 116') du fil métallique central s'étend distalement par rapport au corps tubulaire (140, 140') ou le corps tubulaire présente une ouverture dans une paroi latérale pour permettre un contact de fluide avec le fil métallique central.
